# EUROPEAN PATENT SPECIFICATION

(11) **EP 3 871 630 B1**
(45) Date of publication and mention of the grant of the patent: **06.07.2022**
(21) Application number: 20425007.0
(22) Date of filing: 27.02.2020
(51) Int. Cl.: A61B 90/70, A61B 90/00, A47L 15/00, A61C 19/00

(54) **PROFESSIONAL WASHING APPARATUS COMPRISING A SYSTEM FOR THE AUTOMATIC LINEAR TRANSFER OF INSTRUMENT-CARRYING TROLLEYS AND SAFETY DEVICE INCLUDED IN SAID SYSTEM**
PROFESSIONELLE WASCHVORRICHTUNG MIT EINEM SYSTEM ZUR AUTOMATISCHEN LINEAREN ÜBERTRAGUNG VON INSTRUMENTENTRAGENDEN WAGEN UND SICHERHEITSVORRICHTUNG
APPAREIL DE LAVAGE PROFESSIONNEL COMPRENANT UN SYSTÈME DE TRANSFERT LINÉAIRE AUTOMATIQUE DES CHARIOTS PORTE-INSTRUMENTS ET UN DISPOSITIF DE SÉCURITÉ INCLUS DANS LEDIT SYSTÈME

(43) Date of publication of application: 01.09.2021
(73) Proprietor: Bonferraro S.p.A., 37060 Bonferraro (VR) (IT)
(72) Inventor: Gobbi, Ezio, 46037 Roncoferraro MN (IT); Piazza, Damiano, 37054 Nogara VR (IT)
(74) Representative: Concone, Emanuele

(56) References cited:
- EP-A1- 1 905 343
- EP-A1- 1 949 843
- EP-A1- 3 202 300
- WO-A1-2015/068131

## Description

The present invention concerns a professional washing apparatus suitable for the treatment of surgical instruments, laparoscopes or other medical devices (hereinafter referred to as "instruments") equipped with a system for the automatic loading and unloading along a linear path of instrument-carrying trolleys in a washing chamber, and in particular a safety device included in said system to prevent damage to the operator and to the apparatus itself in case the trolley encounters an obstacle on its path during the transfer to and from the washing chamber. In the following specific reference will be made to an apparatus for washing instruments, but it is clear that the same type of device can be applied to other washing appliances such as dishwashers, disinfection equipment, sterilizers, etc.

It is known that a professional washing apparatus of this type includes a washing chamber with two opposing openings closed by relevant vertically sliding doors to give access to the chamber, so that a trolley with dirty instruments enters the chamber on one side and leaves the chamber through the opposing side after washing. The transfer of the trolley in and out of the washing chamber is carried out automatically by a system substantially comprising two independent mechanisms with reciprocating motion along a linear path extending from a loading end to an unloading end of the apparatus.

In practice, each mechanism essentially consists of a cursor mounted on a linear actuator (e.g. toothed belt, worm screw, piston, etc.) and equipped with a push/pull lever that is mobile between a rest position, where it allows the cursor to pass a trolley without engaging it, and a working position where it engages the trolley to push it into the washing chamber, during loading, and to pull it out of the washing chamber during unloading.

As mentioned above, the apparatus must include a safety device that stops the cursor in the event that the trolley engaged by it encounters an obstacle in its path during the transfer to and from the washing chamber, in order to prevent damage to the operator who could be with a part of his body on the transfer path and to the apparatus itself in the event that any instrument protruding from the trolley prevents it from moving or entering the washing chamber.

In conventional apparatuses, this safety function is carried out by means of an electronic control of the power absorbed by the actuator, but this electronic control is quite expensive and also presents problems of calibration of the intervention threshold. In fact, if the threshold is too low it can cause frequent undesired stoppages of the apparatus when the higher absorption is due only to factors not related to the presence of an obstacle (e.g. a higher trolley load, greater friction of the trolley wheels, etc.), while if the threshold is too high it can result in a stoppage of the trolley when damage has already occurred to the operator or the apparatus due to the obstacle. In addition, the use of an electronic control in an apparatus that produces considerable vibration and is in a generally hot and humid environment due to the steam released when the washing chamber is opened, can result in reliability problems.

The object of the present invention is therefore to provide a washing apparatus that is free from said drawbacks. This object is achieved with a washing apparatus equipped with automatic mechanisms for the loading and unloading of the trolleys in which each cursor is connected to its own actuator through a safety device that allows a relative movement between these two components, along the direction of transfer, when the resistance of an elastic contrast element is exceeded, such relative movement being detected by at least one sensor configured and operatively connected to stop the actuator.

A first important advantage of the present apparatus is to reduce the cost and increase the reliability of the safety device, by replacing electronic components with electro-mechanical components that are cheaper and more robust.

A second significant advantage of this apparatus is that the safety device intervenes only when the cursor actually encounters an obstacle, avoiding the stoppage of the apparatus due to factors unrelated to the presence of an obstacle.

A further significant advantage of the above-mentioned safety device of this washing apparatus stems from its simplicity of calibration, which only requires adjusting the pre-load of the elastic contrast element.

Further advantages and characteristics of the apparatus according to the present invention will be evident to those skilled in the art from the following detailed description of some embodiments thereof with reference to the annexed drawings, wherein:
Fig.1 is a perspective view from above of a trolley transfer mechanism, without its casing to show the internal components, with the cursor in push configuration;
Fig.2 is a view similar to the previous one of a transfer mechanism complete with casing and with the cursor in pull configuration;
Fig.3 is a top plan view of the cursor/actuator group of the mechanism of Fig.1, with the relevant support and guide elements;
Figs.4a-4c are partially sectional views along line A-A of Fig.3 showing only the cursor/actuator group, with the safety device in the three possible operating conditions;
Figs.5a-5c are partially sectional views along line B-B of Fig.4a that correspond to the views in Figs.4a-4c;
Fig.6 is a top perspective view of the cursor/actuator group in the normal operating condition corresponding to Figs.4a, 5a;
Fig.7 is a partial sectional view of the cursor/actuator group of Fig.6 taken along the vertical midplane; and
Figs.8a-8b are schematic views similar to Fig.4a showing only the cursor with the relevant device for the movement of the pushing lever which is represented in the working position and in the rest position respectively.

WO2015068131A1 published on 2015-05-14 discloses a sliding loader trolley for loading medical instruments into a washing chamber.

EP3202300B1 published on 2017-11-22 and EP1949843B1 published on 2013-07-10 disclose safety devices for a closing element of a washing machine.

The invention is defined in appended independent claim 1 and further embodiments are defined in the dependent claims.

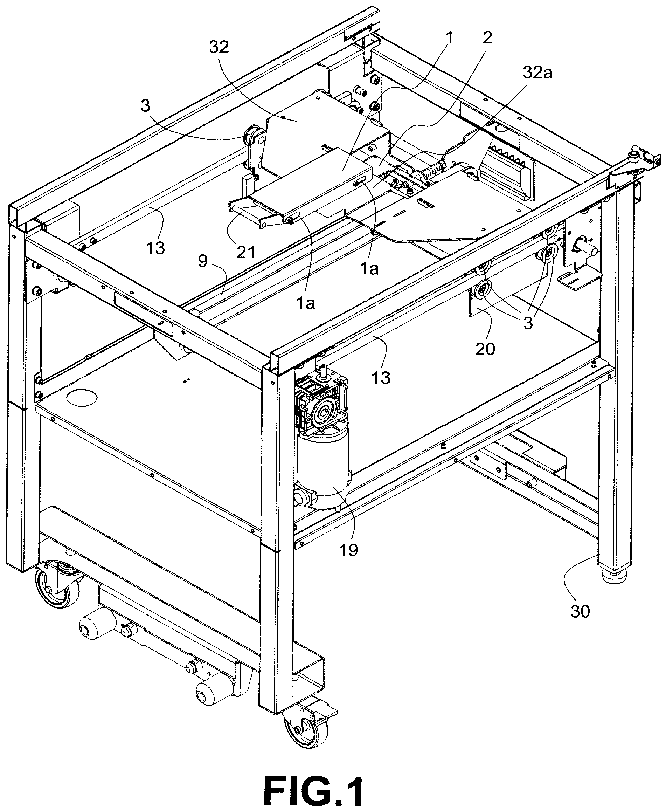

Referring first to Figs. 1-3, there is seen that a transfer mechanism traditionally includes a frame 30 with a substantially parallelepiped shape that carries at a first end a motor 19 that drives a toothed belt 9, arranged longitudinally in a central position. The latter is driven by a transverse transmission shaft 14' connected to a first toothed wheel 10' on which the toothed belt 9 is wound, which at the opposite end of frame 30 is wound on a second toothed wheel 10 carried by an analogous transverse idle shaft 14. The cursor interacting with the instrument-carrying trolleys (not shown) is driven by the toothed belt 9 in such a way as to perform a longitudinal reciprocating motion moving towards and away from the washing chamber (not shown) according to the direction of operation of motor 19.

More specifically, the cursor slides along longitudinal side guides 13 by means of horizontal-axis wheels 3 mounted on vertical side plates 20; in particular, in the example shown there are four wheels 3 for each side arranged in opposing pairs above and below guides 13. Furthermore, the position of the cursor is preferably detected by sensors that inform the mechanism management system about the cursor movement and its stop in the desired positions, so as to precisely control motor 19 in the operating time and direction of rotation.

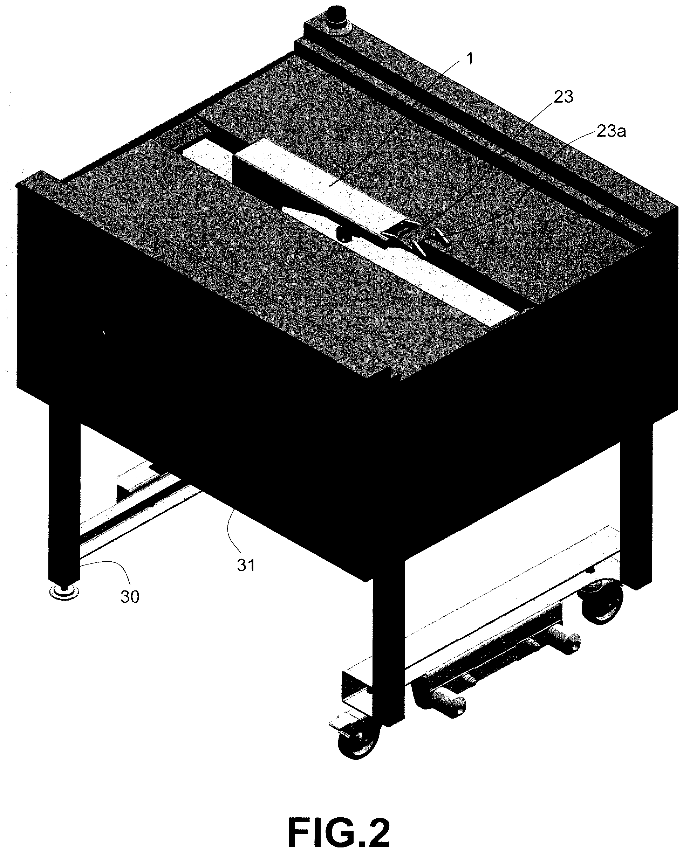

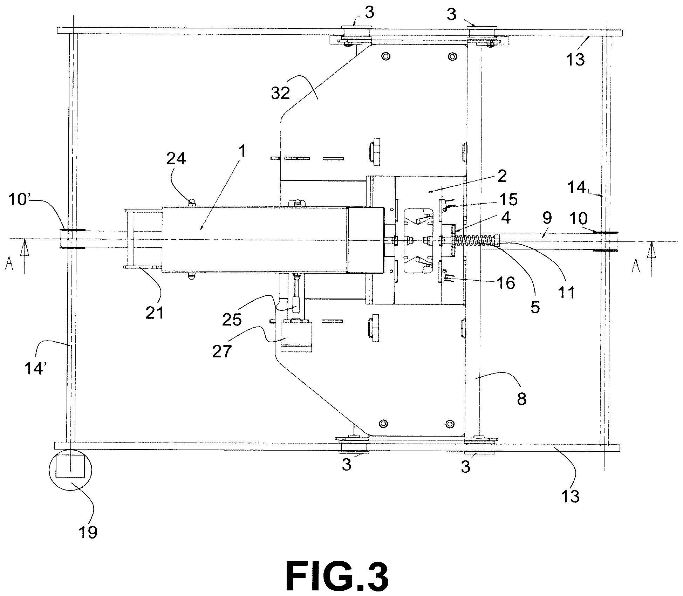

The cursor includes an upper plate 32 that extends horizontally between the vertical side plates 20 and is equipped with a central top turret 32a that carries the lever which engages the instrument-carrying trolley, said turret 32a being protected by a cover 1 that is longitudinally mobile with respect thereto thanks to side slots 1a. Figures 1 and 3 show a push lever 21 to insert the trolley in the washing chamber, the distal end of said lever 21 having a simple straight shape, while Fig.2 shows a pull lever 23 to extract the trolley from the washing chamber and thus equipped with end hooks 23a to engage a bottom crossbar of the trolley.

As shown in Fig.2, frame 30 is fitted in its upper portion with a plurality of cover plates forming a casing 31 to protect the internal components of the mechanism. The only part remaining on the outside is obviously turret 32a, which carries the lever that must interact with the trolley and moves along a central longitudinal lane obtained from the spacing of the two top plates of casing 31.

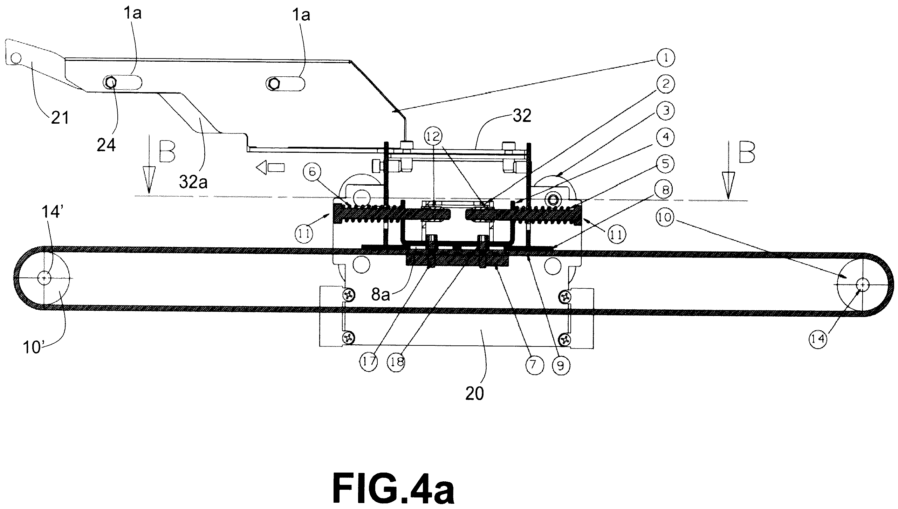

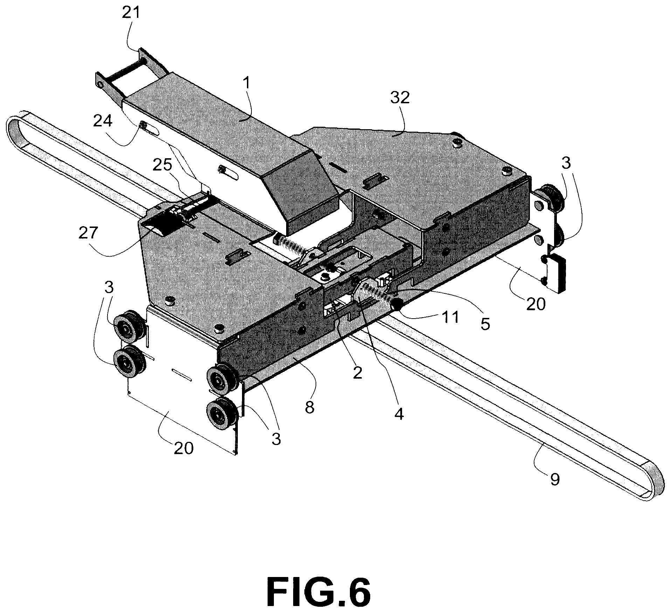

Referring now also to figures 4a, 5a, 6 and 7, there is seen that the innovative aspect of this invention lies in the connection of the cursor to the toothed belt 9 via an electro-mechanical safety device integrated in the cursor. Considering that the front part of the cursor is the one where turret 32a protrudes, the structure of the cursor is composed of the above-mentioned upper horizontal plate 32 connected to a lower horizontal plate 8 through a pair of vertical transverse walls respectively front 22' and rear 22, also extending between the vertical side plates 20.

The safety device comprises a central support 2 which is mounted transversely on the lower plate 8, straddling belt 9, and carries a pair of bushings 12 placed opposite each other along the longitudinal axis of the cursor, corresponding to the midplane of belt 9. These bushings 12 serve as seats for adjusting screws 11 carrying a pair of springs, rear 5 and front 6 respectively, each of which is enclosed between the head of the relevant screw 11 and the vertical outer wall of a deactivation bracket 4 which is essentially U-shaped in longitudinal section.

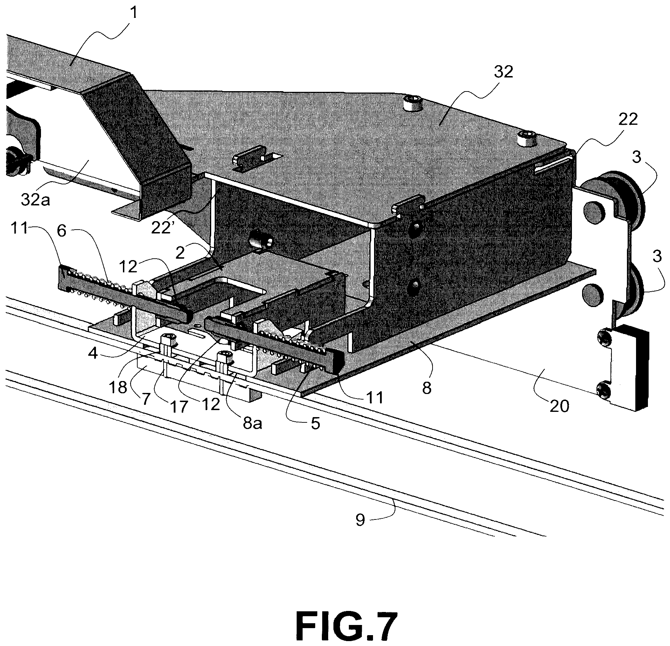

As better shown in Figs.4a and 7, this deactivation bracket 4 extends longitudinally through the central support 2 in a position centered on the longitudinal axis of the cursor, and is attached to belt 9 by means of screws 17 which pass through it and are screwed into a fixing plate 7 located below belt 9. It should be noted that bracket 4 does not come into contact with the lower plate 8, both as a result of spacers 18 placed between the heads of screws 17 and belt 9, and as a result of the vertical distance between the holes through which screws 11 pass and the horizontal portion of bracket 4 through which screws 17 pass. In addition, said lower plate 8 to be crossed by screws 17 is equipped not with simple holes of suitable diameter but with slots 8a elongated in the longitudinal direction, so as to allow a relative movement between bracket 4 and the lower plate 8 as will be described in more detail below.

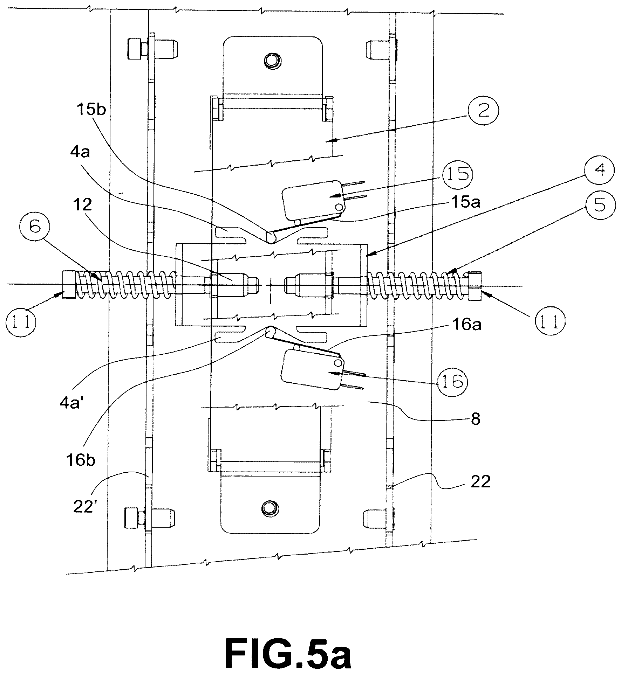

The view of Fig.5a shows the plan profile of bracket 4 whose longitudinal sides 4a, 4a' of the horizontal portion are specularly shaped to interact with two micro-switches 15, 16 mounted on the lower plate 8 in positions adjacent to said sides 4a, 4a'. More specifically, each micro-switch 15, 16 is fitted with a flexible rod 15a, 16a that carries on its free end a rounded protrusion 15b, 16b which in the illustrated "rest" position, i.e. normal operation of the mechanism, is situated at the apex of an outward-facing V shape formed on sides 4a, 4a' respectively adjacent to micro-switch 15, 16 which is not activated in this condition.

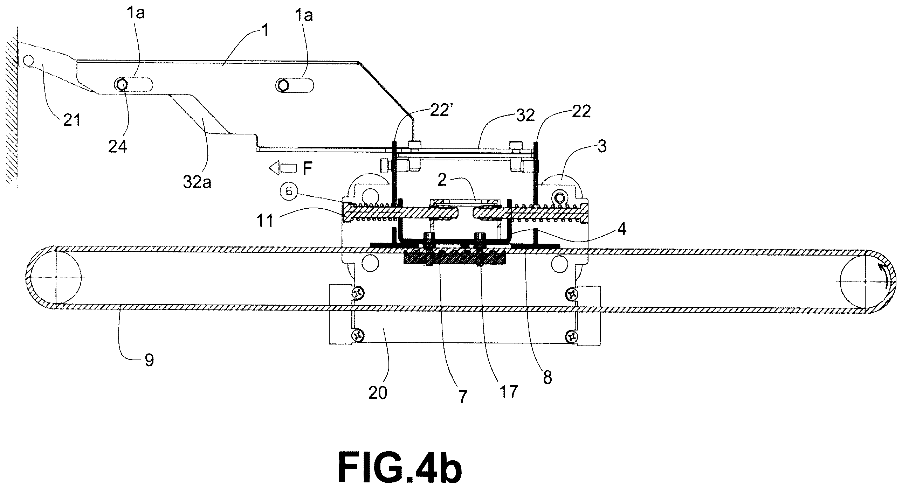

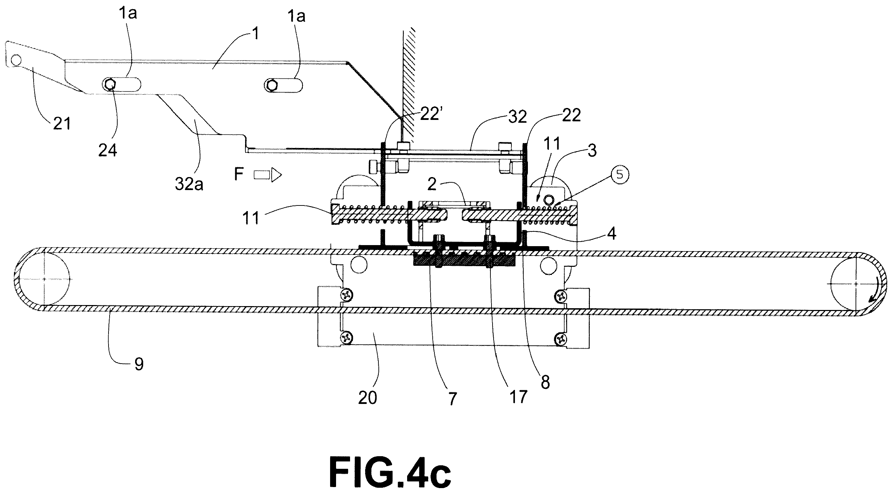

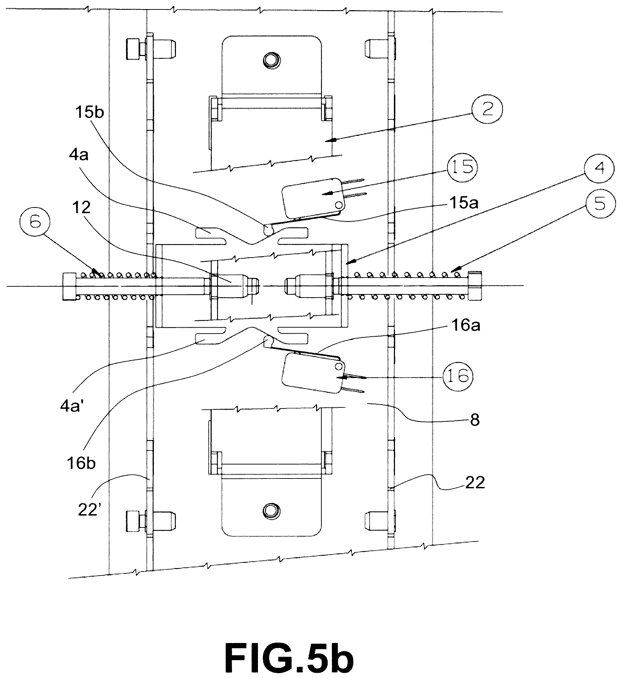

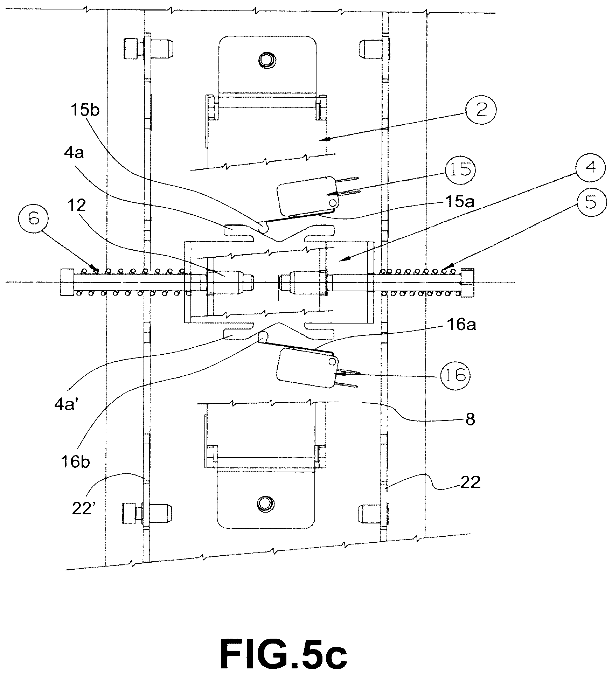

The simple and effective operation of the above safety device integrated in the washing apparatus according to the present invention is now described with reference also to figures 4b, 4c, 5b and 5c.

The first case is illustrated in figures 4b and 5b that concern the contact of the cursor, in particular of lever 21, with an obstacle during the forward run (arrow F). In this condition, even if the cursor is blocked by the obstacle, belt 9 continues to rotate (counterclockwise in the illustrated example) dragging forward the deactivation bracket 4 constrained thereto through screws 17, which slide in slots 8a of the lower plate 8. This relative movement is possible because, as explained above, there is play between plate 8 and bracket 4 which can continue to advance, compressing the front spring 6, until it comes into contact with the front transverse wall 22'.

In practice, however, this contact does not occur because the advancement of bracket 4 implies that protrusions 15b, 16b move away from the longitudinal axis of the cursor following respectively the V shape of walls 4a and 4a', as shown in Fig.5b, with the result of bending rods 15a, 16a which cause the activation of the micro-switches 15, 16 which control respectively the stoppage of motor 19 and the interruption of the power supply to the transfer mechanism. As a consequence, the cursor stops in a very short time, since the stroke of bracket 4 is very short and causes a sure and immediate activation of micro-switches 15, 16. Obviously, the shape and angle of aperture of the V shape can be designed to make the stoppage more or less immediate and to modulate the actuation time/effort.

It should be noted that even just one of the two micro-switches 15, 16 would be sufficient to stop the cursor, but the presence of both is preferable because it is a redundancy that increases the safety of the system. As soon as the obstacle is removed, the compressed spring 6 pushes the cursor forward by pressing on bracket 4 so as to return protrusions 15b, 16b to the "rest" position and deactivate the micro-switches 15, 16 to allow the mechanism to restart.

The second case is illustrated in figures 4c and 5c that concern the contact of the cursor, in particular of turret 32a, with an obstacle during the backward run (arrow F). In this condition, even if the cursor is blocked by the obstacle, belt 9 continues to rotate (clockwise in the illustrated example) dragging backward the deactivation bracket 4 which can continue to move back, compressing the rear spring 5, until it comes into contact with the rear transverse wall 22.

In practice, however, this contact does not occur because the backing of bracket 4 implies that protrusions 15b, 16b move away from the longitudinal axis of the cursor following respectively the V shape of walls 4a and 4a', as shown in Fig.5c, with the result of bending rods 15a, 16a that cause the activation of the micro-switches 15, 16 with an operation completely equal and symmetrical to that of the first case of the forward run.

It is therefore evident that the system according to the present invention guarantees a simple and effective control on the cursor stoppage, by means of inexpensive and robust components that guarantee a high reliability thereof. Also the adjustment of the intervention threshold is particularly easy, since it is sufficient to adjust the pre-compression of the contrast springs 5, 6 which are used to transmit the motion from bracket 4, attached to belt 9, to turret 32a through screws 11 and bushings 12 integral with the central support 2. This adjustment is carried out by screwing or unscrewing screws 11 so as to vary the force required to compress springs 5, 6 to allow the relative movement of bracket 4 with respect to the lower plate 8 as described above.

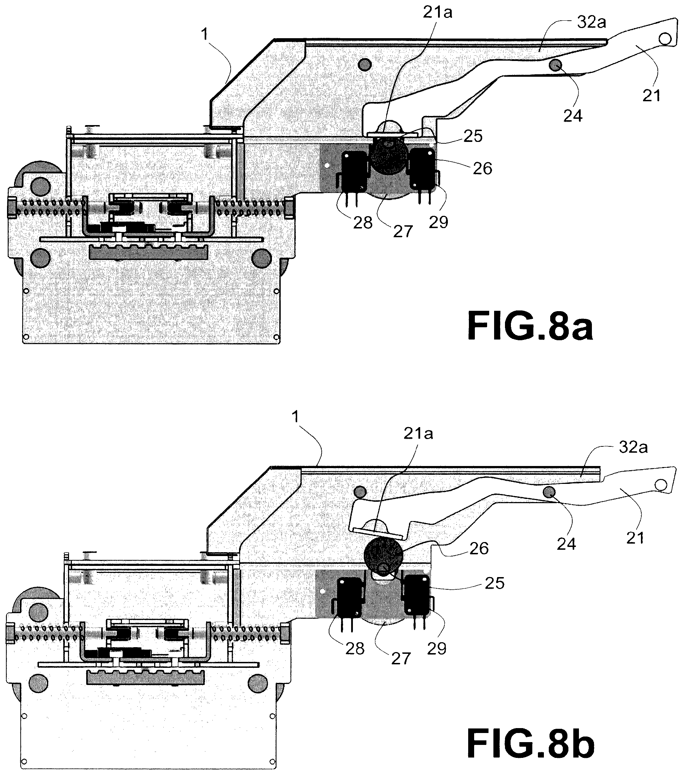

Finally, referring to Figs.8a, 8b there is shown a device to switch the push/pull lever between the rest position and the working position (the figures show the push lever 21 but an identical device is used for the pull lever 23). Lever 21 is pivoted on turret 32a at an axis 24 situated in a forward position such that the weight of the part of lever 21 behind said axis 24 is greater than the weight of the part in front of it. As a consequence, lever 21 spontaneously remains in the working position of Fig.8a, with its front end protruding upwards with respect to the top of turret 32a and its rear end 21a resting on a disc 26 secured in an eccentric position on a transverse shaft 25 which is rotated by a motor 27.

In the condition of Fig.8a, disc 26 extends mainly under shaft 25, which is therefore near the rear end 21a of lever 21, cover 1 being in contact with the front part of lever 21. To move to the rest position shown in Fig.8b, it is sufficient that shaft 25 makes half a turn so that disc 26 extends mainly above shaft 25, whereby it lifts the rear end 21a and causes a rotation (clockwise in the example shown) of lever 21 around axis 24. As a result, the front end of lever 21 is lowered in alignment (or at a lower level) with the top of cover 1, so that the cursor can move underneath the instrument-carrying trolleys without engaging them.

In order to correctly manage the operation of motor 27, the position of disc 26 is preferably detected by means of two micro-switches 28 and 29, with a structure similar to that of micro-switches 15 and 16, which are arranged just below shaft 25 and on the sides thereof, so as to be activated when disc 26 is in the lower position of Fig.8a (also in this case the use of two micro-switches is a redundancy for safety, but only one could be sufficient). In addition, it is possible to manually lock lever 21 or 23 in the rest position of Fig.8b by moving cover 1 forward, so as to lock the rotation around axis 24 even if disc 26 is in the lower position. This allows the trolleys to pass freely over the cursor to manually push/pull them during set-up or maintenance of the mechanism.

It is clear that the embodiment of the apparatus according to the invention described and illustrated above is just an example that is subject to numerous variations, in addition to those already mentioned above. For example, the contrast elastic elements could be different from the simple coil springs 5, 6 (foils, leaf springs, diaphragms, etc.) as long as they perform an equivalent function, and the same applies to the push/pull lever switching device, which instead of the rotating actuator 25-27 could provide a linear actuator (piston, screw, rack, etc.). Similarly, micro-switches 15, 16 could be replaced by other types of sensors (magnetic, optical, potentiometric, etc.) of the relative movement of bracket 4 or could provide a different mechanical configuration (rocker arm, rotating element, etc.), and the same applies to micro-switches 28, 29.

Moreover, it is obvious that the exact shape, arrangement and configuration of the components can vary provided that they are in the scope of the appended claims.

For example, the cursor could be placed sideways to the trolleys instead of below them, in which case there would no longer be the central symmetry described above and the push/pull lever would extend into a vertical plane and its switching would take place with a horizontal movement (in practice the cursor/actuator assembly would be rotated 90° around the longitudinal axis of the mechanism).

## Claims

1. Washing apparatus provided with a system for the automatic loading and unloading of instrument-carrying trolleys in a washing chamber equipped with two opposing openings,
**characterized in that** said system comprises:
two independent mechanisms with reciprocating motion along a linear path extending from a loading end to an unloading end of the apparatus, each of said mechanisms being essentially composed of a cursor mounted on a linear actuator (9) and equipped with a push (21) or pull (23) lever which is mobile between a rest position, in which it allows said cursor to pass an instrument-carrying trolley without engaging it, and a working position in which it engages the instrument-carrying trolley to push it into the washing chamber during loading and to pull it out of the washing chamber during unloading, the apparatus comprising a safety device that stops the cursor in case the instrument-carrying trolley encounters an obstacle in its path, and
said safety device is arranged between the cursor and said linear actuator and includes:
- a deactivation bracket (4) attached to the linear actuator (9) and mobile with respect to the cursor;
- two or more contrast elastic means (5, 6) arranged between said deactivation bracket (4) and the cursor so as to oppose the relative motion between them and to transmit the movement from the linear actuator to the cursor in both directions of said reciprocating motion;
- means of adjustment (11) of the pre-load of said two or more contrast elastic means, so as to determine the threshold of intervention of the safety device;
- one or more sensors (15, 16) configured and operatively connected to detect said relative motion between the deactivation bracket (4) and the cursor and consequently stop the linear actuator.

2. Washing apparatus according to claim 1, **characterized in that** the linear actuator is arranged under the path of the instrument-carrying trolleys, preferably along the longitudinal axis of the cursor, and the cursor performs its reciprocating motion passing under the instrument-carrying trolleys.

3. Washing apparatus according to claim 2, **characterized in that** the linear actuator is arranged along the longitudinal axis of the cursor and the safety device includes a central support (2) which is mounted transversely on a lower plate (8) of the trolley, straddling the linear actuator, and carries a pair of bushings (12) arranged in opposing position along the longitudinal axis of the mechanism which act as seats for adjusting screws (11) carrying a pair of springs (5, 6) which perform the function of said contrast elastic means, each of said springs (5, 6) being enclosed between the head of the relevant adjusting screw (11) and the vertical outer wall of the deactivation bracket (4) which has a substantially U-shaped longitudinal section.

4. Washing apparatus according to claim 3, **characterized in that** the deactivation bracket (4) extends longitudinally through the central support (2) in a position centered on the longitudinal axis of the cursor, and is fixed to the linear actuator by means of screws (17) which pass through slots (8a) elongated in the longitudinal direction formed in the lower plate (8).

5. Washing apparatus according to claim 3 or 4, **characterized in that** the plan profile of the deactivation bracket (4) includes two longitudinal sides (4a, 4a') shaped in a specular way to interact with two micro-switches (15, 16) mounted on the lower plate (8) in positions adjacent to said longitudinal sides (4a, 4a'), so as to perform the function of said sensors of the relative motion between the deactivation bracket (4) and the cursor.

6. Washing apparatus according to claim 5, **characterized in that** each micro-switch (15, 16) is provided with a flexible rod (15a, 16a) carrying on its free end a rounded protrusion (15b, 16b) which, in the normal operating position of the mechanism, is situated at the apex of an outward-facing V shape formed on the longitudinal side (4a, 4a') respectively adjacent to the micro-switch (15, 16), which in this condition is not activated.

7. Washing apparatus according to any of the preceding claims, **characterized in that** the linear actuator includes a motor (19), placed at a first end of the mechanism, which drives a toothed belt (9) through a transverse drive shaft (14') connected to a first toothed wheel (10') on which said toothed belt (9) is wound, which at the opposite end of the mechanism is wound on a second toothed wheel (10) carried by a transverse idle shaft (14).

8. Washing apparatus according to any of the preceding claims, **characterized in that** the push lever (21) and the pull lever (23) are each hinged on the cursor at an axis (24) located in an advanced position such that the weight of the part of the lever (21, 23) which is behind said axis (24) is greater than the weight of the part in front of it, so that the lever (21, 23) remains spontaneously raised in the working position with the front end protruding upwards with respect to the cursor structure in the position of engagement of an instrument-carrying trolley.

9. Washing apparatus according to claim 8, **characterized in that** the rear end of the lever (21, 23) rests on a disc (26) secured in an eccentric position on a rotating transverse shaft (25), said disc (26) extending mainly below the shaft (25) when the lever (21, 23) is in the working position, the switching of the lever (21, 23) to the rest position being performed by a motor (27) rotating said shaft (25) by half a turn so that the disc (26) extends mainly above the shaft (25), so as to lift the rear end of the lever (21, 23) and cause it to rotate around the axis (24) whereby the front end of the lever (21, 23) lowers in alignment with or below the top of the cursor structure.

10. Washing apparatus according to claim 9, **characterized in that** two micro-switches (28, 29) are placed just under the shaft (25) and on its sides, so that they are activated when the disc (26) is in the position below the shaft (25).

11. Washing apparatus according to any of the preceding claims, **characterized in that** the cursor slides along longitudinal side guides (13) by means of horizontal-axis wheels (3) mounted on vertical side plates (20), preferably four wheels (3) for each side arranged in opposing pairs above and below said guides (13).

12. Washing apparatus according to claim 11, **characterized in that** the cursor comprises a top turret (32a) carrying the lever (21, 23) which engages the instrument-carrying trolley, said turret (32a) extending centrally from an upper plate (32) that extends between the vertical side plates (20) and is connected to the lower plate (8) through a pair of vertical transverse walls (22, 22') also extending between the vertical side plates (20).

13. Washing apparatus according to claim 12, **characterized in that** the top turret (32a) is protected by a cover (1) which is longitudinally movable with respect thereto between a rearmost position, in which it allows the switching of the push (21) or pull (23) lever between the rest position and the working position, and a forward position in which it locks the push (21) or pull (23) lever in the rest position.

## Patentansprüche

1. Waschvorrichtung, versehen mit einem System zum automatischen Beladen und Entladen von Instrumente tragenden Wagen in eine Waschkammer, die mit zwei gegenüber liegenden Öffnungen ausgerüstet ist,
**dadurch gekennzeichnet, dass** das besagte System umfasst:
zwei unabhängige Mechanismen mit hin- und her gehender Bewegung entlang eines linearen Pfads, der sich von einem Beladungsende bis zu einem Entladungsende der Vorrichtung erstreckt, wobei jeder der besagten Mechanismen substantiell zusammengesetzt ist aus einem Läufer, welcher an einem linearen Aktuator (9) montiert und mit einem Druckhebel (21) oder Zughebel (23) ausgerüstet ist, der zwischen einer Ruheposition, in welcher er es dem besagten Läufer erlaubt, einen Instrumente tragenden Wagen passieren zu lassen, ohne sich an ihm einzukuppeln, und einer Arbeitsposition bewegbar ist, in welcher er sich an dem Instrumente tragenden Wagen einkuppelt, um jenen während der Beladung in die Waschkammer hinein zu schieben, und um jenen während der Entladung aus der Waschkammer heraus zu ziehen, wobei die Vorrichtung eine Sicherheitseinrichtung aufweist, die den Läufer stoppt, falls der Instrumente tragende Wagen auf seinem Weg auf ein Hindernis trifft, und wobei die besagte Sicherheitseinrichtung zwischen dem Läufer und dem besagten linearen Aktuator angeordnet ist und umfasst:
- eine Deaktivierungsklammer (4), die an dem linearen Aktuator (9) angeschlossen ist und in Bezug auf den Läufer mobil ist;
- zwei oder mehr, einander gegenüber stehende, elastische Mittel (5, 6), die zwischen der besagten Deaktivierungsklammer (4) und dem Läufer angeordnet sind, um sich der relativen Bewegung zwischen jenen zu widersetzen und die Bewegung des linearen Aktuators in beiden Richtungen der besagten hin und her gehenden Bewegung auf den Läufer zu übertragen;
- Mittel (11) zum Einstellen der Vorspannung der besagten zwei oder mehr, einander gegenüber stehenden, elastischen Mittel, um so die Schwelle des Einschreitens der Sicherheitseinrichtung zu bestimmen;
- einen oder mehrere Sensoren (15, 16), die dazu ausgelegt und wirkungsmäßig angeschlossen sind, um die besagte Relativbewegung zwischen der Deaktivierungsklammer (4) und dem Läufer zu erkennen und infolgedessen den linearen Aktuator zu stoppen.

2. Waschvorrichtung gemäß Anspruch 1, **dadurch gekennzeichnet, dass** der lineare Aktuator unter dem Pfad der Instrumente tragenden Wagen angeordnet ist, vorzugsweise entlang der Längsachse des Läufers, und dass der Läufer seine hin- und her gehende Bewegung unter den Instrumente tragenden Wagen ausführt.

3. Waschvorrichtung gemäß Anspruch 2, **dadurch gekennzeichnet, dass** der lineare Aktuator entlang der Längsachse des Läufers angeordnet ist, und dass die Sicherheitseinrichtung eine mittige Stütze (2) umfasst, die an einer unteren Platte (8) des Wagens quer montiert ist, den linearen Aktuator überbrückend, und die ein Paar von Büchsen (12) trägt, welche an einer gegenüber liegenden Position entlang der Längsachse des Mechanismus angeordnet sind, die als Aufnahmen für Einstellschrauben (11) dienen, die ein Paar von Federn (5, 6) tragen, welche die Funktion der besagten, einander gegenüber stehenden, elastischen Mittel ausführen, wobei jede der besagten Federn (5, 6) eingeschlossen ist zwischen dem Kopf der entsprechenden Einstellschraube (11) und der vertikalen Außenwand der Deaktivierungsklammer (4), welche einen substantiell U-förmigen Längsschnitt aufweist.

4. Waschvorrichtung gemäß Anspruch 3, **dadurch gekennzeichnet, dass** die Deaktivierungsklammer (4) sich durch die mittige Stütze (2) in Längsrichtung hindurch erstreckt an einer Position, die auf der Längsachse des Läufers zentriert ist, und an dem linearen Aktuator befestigt ist mittels Schrauben (17), welche durch Schlitze (8a) hindurch laufen, die in der Längsrichtung gestreckt in der unteren Platte (8) eingeformt sind.

5. Waschvorrichtung gemäß Anspruch 3 oder 4, **dadurch gekennzeichnet, dass** die Draufsicht auf die Deaktivierungsklammer (4) zwei Längsseiten (4a, 4a') aufweist, welche spiegelbildlich geformt sind, um mit zwei Mikroschaltern (15, 16) zusammenzuwirken, die an der unteren Platte (8) montiert sind an Positionen nahe bei den besagten Längsseiten (4a, 4a'), um so die Funktion der besagten Sensoren für eine relative Bewegung zwischen der Deaktivierungsklammer (4) und dem Läufer zu übernehmen.

6. Waschvorrichtung gemäß Anspruch 5, **dadurch gekennzeichnet, dass** jeder Mikroschalter (15, 16) mit einem biegsamen Stab (15a, 16a) versehen ist, der an seinem freien Ende eine abgerundete Ausstülpung (15b, 16b) aufweist, die sich in der normalen Arbeitsposition der Mechanik am Scheitelpunkt einer nach außen gerichteten V-Form befindet, die an der Längsseite (4a, 4a') ausgeformt ist, jeweils nächstliegend zu demjenigen Mikroschalter (15, 16) ist, welcher in diesem Zustand nicht aktiviert ist.

7. Waschvorrichtung gemäß einem der vorhergehenden Ansprüche, **dadurch gekennzeichnet, dass** der lineare Aktuator einen an einem ersten Ende der Mechanik angeordneten Motor (19) umfasst, der einen Zahnriemen (9) antreibt über eine quer verlaufende Antriebswelle (14'), die an einem ersten Zahnrad (10') angeschlossen ist, um welches der besagte Zahnriemen (9) gewunden ist, der an dem entgegengesetzten Ende der Mechanik um ein zweites Zahnrad (10) gewunden ist, das von einer quer verlaufenden, leerlaufenden Welle (14) getragen wird.

8. Waschvorrichtung gemäß einem der vorhergehenden Ansprüche, **dadurch gekennzeichnet, dass** der Druckhebel (21) und der Zughebel (23) jeweils an dem Läufer an einer Achse (24) gelenkig gelagert ist, die sich an einer vorgeschobenen Position befindet, so dass das Gewicht desjenigen Teils des Hebels (21, 23), der sich hinter der besagten Achse (24) befindet, größer ist als das Gewicht desjenigen Teils, der sich vor ihr befindet, so dass der Hebel (21, 23) in der Arbeitsposition spontan angehoben bleibt, wobei das vordere Ende bezüglich der Läuferstruktur an der Position des Einrastens eines Instrumente tragenden Wagens nach oben herausragt.

9. Waschvorrichtung gemäß Anspruch 8, **dadurch gekennzeichnet, dass** das rückwärtige Ende des Hebels (21, 23) auf einer Scheibe (26) ruht, die in einer exzentrischen Position an einer rotierenden, quer verlaufenden Welle (25) befestigt ist, wobei die besagte Scheibe (26) sich größtenteils unterhalb der Welle (25) erstreckt, wenn sich der Hebel (21, 23) in der Arbeitsposition befindet, wobei das Umschalten des Hebels (21, 23) in die Ruheposition von einem Motor (27) ausgeführt wird, der die besagte Welle (25) um eine halbe Umdrehung rotiert, so dass die Scheibe (26) sich größtenteils oberhalb der Welle (25) erstreckt, wodurch das rückwärtige Ende des Hebels (21, 23) angehoben wird und veranlasst wird, um die Achse (24) zu rotieren, wobei das vordere Ende des Hebels (21, 23) abgesenkt wird in einer Ausrichtung mit dem oberen Ende oder unterhalb des oberen Endes der Läuferstruktur.

10. Waschvorrichtung gemäß Anspruch 9, **dadurch gekennzeichnet, dass** zwei Mikroschalter (28, 29) geradewegs unterhalb der Welle (25) sowie an deren Seiten angeordnet sind, so dass sie aktiviert werden, wenn die Scheibe (26) sich in der Position unterhalb der Welle (25) befindet.

11. Waschvorrichtung gemäß einem der vorhergehenden Ansprüche, **dadurch gekennzeichnet, dass** der Läufer entlang longitudinaler Seitenführungen (13) gleitet mittels Laufrädern (3) an horizontalen Achsen, die an vertikalen Seitenplatten (20) montiert sind, vorzugsweise mittels vier Laufrädern (3) an jeder Seite, die in Form von einander gegenüber liegenden Paaren oberhalb und unterhalb der besagten Führungen (13) angeordnet sind.

12. Waschvorrichtung gemäß Anspruch 11, **dadurch gekennzeichnet, dass** der Läufer einen den Hebel (21, 23) tragenden, oberen Turm (32a) aufweist, der mit dem Instrumente tragenden Wagen in Eingriff kommt, wobei der besagte Turm (32a) sich mittig aus einer oberen Platte (32) erhebt, die sich zwischen den vertikalen Seitenplatten (20) erstreckt und an der unteren Platte (8) angeschlossen ist über ein Paar von vertikalen Querwänden (22, 22'), die sich ebenfalls zwischen den vertikalen Seitenplatten (20) erstrecken.

13. Waschvorrichtung gemäß Anspruch 12, **dadurch gekennzeichnet, dass** der obere Turm (32a) durch eine Abdeckung (1) geschützt ist, die in Bezug dazu in Längsrichtung bewegbar ist zwischen einer hintersten Position, in der sie die Umschaltung eines Druck- (21) oder Zughebels (23) zwischen der Ruheposition und der Arbeitsposition erlaubt, und einer vorderen Position, in der sie den Druck- (21) oder Zughebel (23) in die Ruheposition verriegelt.

## Revendications

1. Appareil de lavage prévu avec un système pour le chargement et le déchargement automatique de chariots porte-instruments dans une chambre de lavage équipée de deux ouvertures opposées, **caractérisé en ce que** ledit système comprend :
deux mécanismes indépendants avec un mouvement de va-et-vient le long d'une trajectoire linéaire s'étendant d'une extrémité de chargement à une extrémité de déchargement de l'appareil, chacun desdits mécanismes étant essentiellement composé d'un curseur monté sur un actionneur linéaire (9) et équipé avec un levier de poussée (21) ou de traction (23) qui est mobile entre une position de repos, dans laquelle il permet audit curseur de faire passer un chariot porte-instruments sans le mettre en prise, et une position de travail dans laquelle il met en prise le chariot porte-instruments pour le pousser dans la chambre de lavage pendant le chargement et l'extraire de la chambre de lavage pendant le déchargement, l'appareil comprenant un dispositif de sécurité qui arrête le curseur dans le cas dans lequel le chariot porte-instruments rencontre un obstacle sur sa trajectoire, et
ledit dispositif de sécurité est agencé entre le curseur et ledit actionneur linéaire et comprend :
- une console de désactivation (4) fixée sur l'actionneur linéaire (9) et mobile par rapport au curseur ;
- deux moyens élastiques de contraste ou plus (5, 6) agencés entre ladite console de désactivation (4) et le curseur afin de s'opposer au mouvement relatif entre eux et afin de transmettre le mouvement de l'actionneur linéaire au curseur dans les deux directions dudit mouvement de va-et-vient ;
- des moyens d'ajustement (11) de la précharge desdits deux moyens élastiques de contraste ou plus, afin de déterminer le seuil d'intervention du dispositif de sécurité ;
- un ou plusieurs capteurs (15, 16) configurés et raccordés, de manière opérationnelle, pour détecter ledit mouvement relatif entre la console de désactivation (4) et le curseur et par conséquent d'arrêter l'actionneur linéaire.

2. Appareil de lavage selon la revendication 1, **caractérisé en ce que** l'actionneur linéaire est agencé sous la trajectoire des chariots porte-instruments, de préférence le long de l'axe longitudinal du curseur, et le curseur réalise son mouvement de va-et-vient passant sous les chariots porte-instruments.

3. Appareil de lavage selon la revendication 2, **caractérisé en ce que** l'actionneur linéaire est agencé le long de l'axe longitudinal du curseur et le dispositif de sécurité comprend un support central (2) qui est monté, de manière transversale, sur une plaque inférieure (8) du chariot, à cheval sur l'actionneur linéaire, et porte une paire de douilles (12) agencées dans la position opposée le long de l'axe longitudinal du mécanisme qui servent de sièges pour des vis de réglage (11) portant une paire de ressorts (5, 6) qui réalise la fonction desdits moyens élastiques de contraste, chacun desdits ressorts (5, 6) étant enfermé entre la tête de la vis de réglage (11) en question et la paroi externe verticale de la console de désactivation (4) qui a une section longitudinale sensiblement en forme de U.

4. Appareil de lavage selon la revendication 3, **caractérisé en ce que** la console de désactivation (4) s'étend de manière longitudinale à travers le support central (2) dans une position centrée sur l'axe longitudinal du curseur, et est fixée sur l'actionneur linéaire au moyen de vis (17) qui passent à travers des fentes (8a) allongées dans la direction longitudinale, formées dans la plaque inférieure (8).

5. Appareil de lavage selon la revendication 3 ou 4, **caractérisé en ce que** le profil plan de la console de désactivation (4) comprend deux côtés longitudinaux (4a, 4a') formés d'une manière spéculaire pour interagir avec deux micro-commutateurs (15, 16) montés sur la plaque inférieure (8) dans des positions adjacentes auxdits côtés longitudinaux (4a, 4a'), afin de réaliser la fonction desdits capteurs du mouvement relatif entre la console de désactivation (4) et le curseur.

6. Appareil de lavage selon la revendication 5, **caractérisé en ce que** chaque micro-commutateur (15, 16) est prévu avec une tige flexible (15a, 16a) portant, sur son extrémité libre, une saillie arrondie (15b, 16b) qui, dans la position de fonctionnement normale du mécanisme, est située au sommet d'une forme de V orientée vers l'extérieur, formée sur le côté longitudinal (4a, 4a') respectivement adjacent au micro-commutateur (15, 16) qui, dans cette condition, n'est pas activé.

7. Appareil de lavage selon l'une quelconque des revendications précédentes, **caractérisé en ce que** l'actionneur linéaire comprend un moteur (19), placé au niveau d'une première extrémité du mécanisme, qui entraîne une courroie dentée (9) par le biais d'un arbre d'entraînement transversal (14') raccordé à une première roue dentée (10') sur laquelle ladite courroie dentée (9) est enroulée, qui, au niveau de l'extrémité opposée du mécanisme, est enroulée sur une seconde roue dentée (10) portée par un arbre intermédiaire transversal (14).

8. Appareil de lavage selon l'une quelconque des revendications précédentes, **caractérisé en ce que** le levier de poussée (21) et le levier de traction (23) sont chacun articulés sur le curseur au niveau d'un axe (24) positionné dans une position avancée de sorte que le poids de la partie du levier (21, 23) qui est derrière ledit axe (24), est supérieur au poids de la partie en face de ce dernier, de sorte que le levier (21, 23) reste spontanément levé dans la position de travail avec l'extrémité avant en saillie vers le haut par rapport à la structure de curseur dans la position de mise en prise d'un chariot porte-instruments.

9. Appareil de lavage selon la revendication 8, **caractérisé en ce que** l'extrémité arrière du levier (21, 23) s'appuie sur un disque (26) fixé dans une position excentrique sur un arbre transversal rotatif (25), ledit disque (26) s'étendant principalement au-dessous de l'arbre (25) lorsque le levier (21, 23) est dans la position de travail, la commutation du levier (21, 23) dans la position de repos, étant réalisée par un moteur (27) faisant tourner ledit arbre (25) d'un demi-tour de sorte que le disque (26) s'étend principalement au-dessus de l'arbre (25), afin de lever l'extrémité arrière du levier (21, 23) et l'amener à tourner autour de l'axe (24), moyennant quoi l'extrémité avant du levier (21, 23) s'abaisse en alignement avec ou au-dessous de la partie supérieure de la structure de curseur.

10. Appareil de lavage selon la revendication 9, **caractérisé en ce que** deux micro-commutateurs (28, 29) sont placés juste sous l'arbre (25) et sur ses côtés, de sorte qu'ils sont activés lorsque le disque (26) est dans la position au-dessous de l'arbre (25).

11. Appareil de lavage selon l'une quelconque des revendications précédentes, **caractérisé en ce que** le curseur coulisse le long des guides latéraux longitudinaux (13) au moyen de roues d'axe horizontal (3) montées sur des plaques latérales verticales (20), de préférence quatre roues (3) pour chaque côté, agencées en paires opposées au-dessus et au-dessous desdits guides (13).

12. Appareil de lavage selon la revendication 11, **caractérisé en ce que** le curseur comprend une tourelle supérieure (32a) portant le levier (21, 23) qui met en prise le chariot porte-instruments, ladite tourelle (32a) s'étendant de manière centrale à partir d'une plaque supérieure (32) qui s'étend entre les plaques latérales verticales (20) et est raccordée à la plaque inférieure (8) par le biais d'une paire de parois transversales verticales (22, 22') s'étendant également entre les plaques latérales verticales (20).

13. Appareil de lavage selon la revendication 12, **caractérisé en ce que** la tourelle supérieure (32a) est protégée par un couvercle (1) qui est longitudinalement mobile par rapport à cette dernière entre la position située le plus en arrière dans laquelle elle permet la commutation du levier de poussée (21) ou de traction (23) entre la position de repos et la position de travail, et une position avant dans laquelle elle bloque le levier de poussée (21) ou de traction (23) dans la position de repos.
